# EUROPÄISCHE PATENTSCHRIFT

(11) **EP 1 644 066 B1**
(45) Veröffentlichungstag und Bekanntmachung des Hinweises auf die Patenterteilung: **16.07.2008**
(21) Anmeldenummer: 04740797.8
(22) Anmeldetag: 08.07.2004
(51) Int. Cl.: A61M 25/00

(54) **BALLONKATHETER**
BALLOON CATHETER
CATHETER A BALLON

(30) Priorität: 16.07.2003 DE 10332251
(43) Veröffentlichungstag der Anmeldung: 12.04.2006
(73) Patentinhaber: B. BRAUN MELSUNGEN AG, 34212 Melsungen (DE)
(72) Erfinder: WÖBKEN, Henning, 12305 Berlin (DE); VAN HUYNH, Qui, 14057 Berlin (DE); CALISSE, Jorge, 12203 Berlin (DE)
(74) Vertreter: Selting, Günther
(86) Internationale Anmeldenummer: PCT/EP2004/007493
(87) Internationale Veröffentlichungsnummer: WO 2005/007227

(56) Entgegenhaltungen:
- EP-A- 0 821 979
- WO-A-92/03178
- US-A- 5 410 797

## Beschreibung

Die Erfindung betrifft einen Ballonkatheter mit einem schlauchförmigen proximalen Schaft, einem ein Führungsdrahtlumen enthaltenden schlauchförmigen distalen Schaft und einem den proximalen und den distalen Schaft verbindenden Übergangsabschnitt, an dem ein Ende des Führungsdrahtlumens seitlich austritt.

Für die Behandlung von Blutgefäßen des Vascularsystems werden PTCA-Katheter eingesetzt (Percutaneous Transluminal Coronary Angioplasty). Hierbei handelt es sich um Ballonkatheter, die über einen Führungsdraht durch die Krümmungen des Vascularsystems an den Zielort geschoben werden. EP 0 821 979 A2 (SciMed) beschreibt ein PTCA-Katheter, durch den der Führungsdraht über die gesamte Katheterlänge hindurchgeht. Zum Wechseln des Katheters muss dieser über seine gesamte Länge von dem Führungsdraht abgezogen und durch einen neuen Katheter ersetzt werden.

WO 92/03178 (SciMed) beschreibt einen PTCA-Katheter, der aus einem relativ steifen längeren proximalen Schaft und einem relativ weichen kürzeren distalen Schaft besteht, an dem sich der Ballon befindet. Ein Führungsdrahtlumen ist nur in dem distalen Schaft vorgesehen. Das proximale Austrittsende des Führungsdrahtlumens befindet sich im Bereich des Übergangsabschnittes. Es wird also nur der distale Schaft über den Führungsdraht geschoben. Im Übergangsabschnitt kann eine Verstärkung in der Außenwand des Katheters und/oder an dem Führungsdrahtlumen vorgesehen sein.

Der Übergangsbereich zwischen dem steifen proximalen und dem flexiblen distalen Schaft ist entweder knickanfällig oder zu steif oder er weist einen zu hohen Außendurchmesser auf. Der Kathether muss gerade im Übergangsabschnitt knickfest sein und hohe Vorschubkräfte übertragen können.

Der Erfindung liegt die Aufgabe zugrunde, einen Ballonkatheter mit im Übergangsabschnitt austretendem Führungsdrahtlumen zu schaffen, der diesen Anforderungen nach hoher Knickstabilität, hoher Flexibilität, guter Kraftübertragung und geringsten Außenabmessungen gerecht wird.

Die Erfindung ist durch den Patentanspruch 1 definiert. Hiernach enthält der Übergangsabschnitt zwischen dem proximalen und dem distalen Schaft eine Drahtwendel, die mit einem Versteifungsdraht verbunden ist, wobei der Versteifungsdraht die Drahtwendel überragt und an einem Ende bis in den proximalen Schaft und am anderen Ende bis in den Bereich des Führungsdrahtlumens ragt. Die Drahtwendel stützt den Übergangsbereich von innen und schützt diesen damit vor Knickung, wobei die vorzugsweise aneinanderliegenden Windungen für eine gute Kraftübertragung sorgen. Der in der Wendel befindliche Versteifungsdraht sorgt für einen guten Steifigkeitsübergang vom proximalen Schaft über die Drahtwendel hinaus bis hinter den Drahtausgang in distaler Richtung, also zum Ballon hin. Der Ballonkatheter, der vorzugsweise ein Rapid-Exchange-PTCA-Ballonkatheter ist, erfüllt hinsichtlich Knickstabilität, Vorschubfähigkeit, Steifigkeit und Flexibilität die gestellten Anforderungen. Er kann darüber hinaus mit geringem Außendurchmesser hergestellt werden.

Vorzugsweise liegen die Windungen der Drahtwendel im unbelasteten Zustand gegeneinander. Die Vorschubkraft wird in Längsrichtung des Katheters von Windung zu Windung übertragen. Die Steifigkeit kann durch eine die Drahtwendel eng umgebende Hülle eingestellt werden.

Gemäß einer bevorzugten Ausgestaltung der Erfindung ist die Drahtwendel an ihrem einen Ende mit dem Führungsdraht verschweißt. Die Drahtwendel kann hohl sein und ein durchgehendes Katheterlumen begrenzen und außen eine abdichtende Hülle tragen. Dabei ist eine Innenhülle in der Drahtwendel nicht vorhanden, wodurch die Außenabmessungen des Katheters im Übergangsbereich verkleinert werden können. Die Hülle, die die Drahtwendel umgibt, kann aus einem Schrumpfschlauch bestehen. Durch Auswahl des Materials und der Wandstärke des Schrumpfschlauchs kann die Steifigkeit der Drahtwendel variiert werden.

Der Versteifungsdraht hat eine begrenzte Länge. Er ist wesentlich kürzer als der Katheter, so dass er jeweils ein Stück weit in den proximalen Schaft und den distalen Schaft hereinragt. Der Versteifungsdraht wird durch die Verbindung mit der Drahtwendel an seinem Platz gehalten. Diese Verbindung erfolgt vorzugsweise durch Anschweißen.

Der Versteifungsdraht liegt an derjenigen Seite an der Drahtwendel an, die der Seite des Austritts des Führungsdrahtlumens abgewandt ist. Auf diese Weise kann der Versteifungsdraht einer im Wesentlichen durchgehenden geraden Wand des Katheters folgen.

Im Folgenden wird unter Bezugnahme auf die Zeichnungen ein bevorzugtes Ausführungsbeispiel der Erfindung näher erläutert. Die Erläuterungen sind nicht so zu verstehen, dass sie den Schutzbereich des Patents einschränken. Dieser wird vielmehr durch die Patentansprüche bestimmt.

Es zeigen:
- Fig. 1: einen Längsschnitt durch einen Teil des Ballonkatheters,
- Fig. 2: einen Schnitt entlang der Linie II-II von Figur 1,
- Fig. 3: einen Schnitt entlang der Linie III-III von Figur 1,
- Fig. 4: einen Schnitt entlang der Linie IV-IV von Figur 1, und
- Fig. 5: einen Schnitt entlang der Linie V-V von Figur 1.

Der dargestellte Katheter ist von seiner Funktion und seinem grundsätzlichem Aufbau her ähnlich wie derjenige, der in WO 92/03178 beschrieben ist. Er weist einen proximalen Schaft 10 auf aus einem flexiblen Rohr 11, das hier einwandig ausgebildet ist. Ferner weist der Katheter einen distalen Schaft 12 auf, der ebenfalls aus einem einwandigen Rohr 13 besteht. Der distale Schaft 12 bildet den patientenseitigen Teil, an dem sich ein (nicht dargestellter) Ballon befindet, der zur Dilatation eines Blutgefäßes aufgepumpt werden kann. Das Katheterlumen 14 geht über die gesamte Länge des Katheters hindurch und ist mit dem Ballon verbunden.

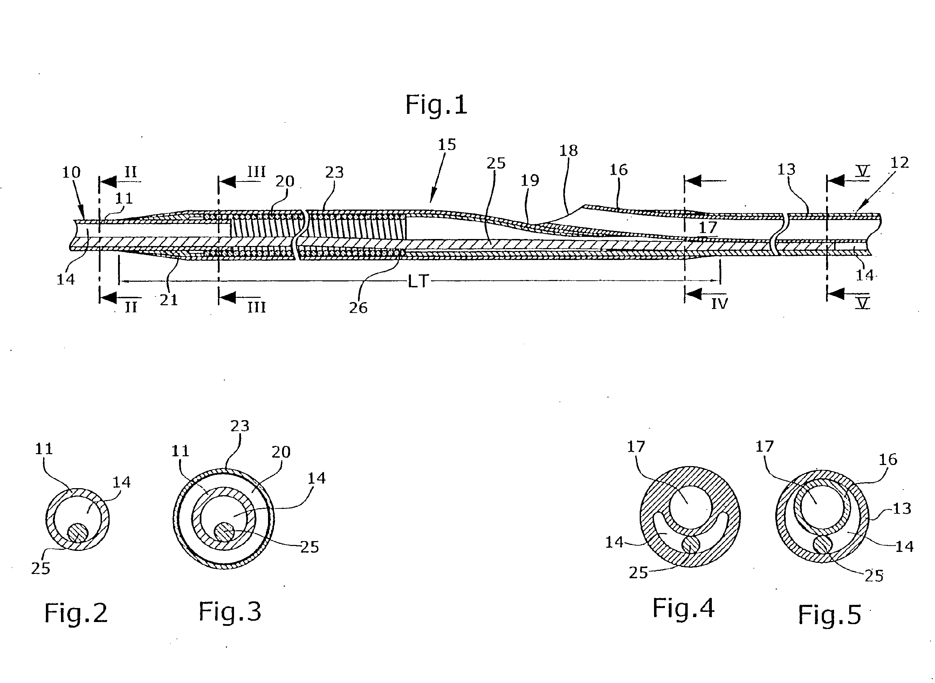

Zwischen dem proximalen und dem distalen Abschnitt befindet sich ein Übergangsabschnitt 15, dessen Länge in Figur 1 mit LT bezeichnet ist. Durch den distalen Schaft 12 erstreckt sich ein flexibles Rohr 16, welches das Führungsdrahtlumen 17 bildet, durch das sich der (nicht dargestellte) Führungsdraht erstreckt, über den der distale Schaft geschoben wird. Das distale Austrittsende 18 des Führungsdrahtlumens 17 befindet sich im Bereich des Übergangsabschnitts 15. Das Rohr 16, das innerhalb des Rohres 13 verläuft, ist durch eine seitliche Öffnung des Rohres 13 schräg herausgeführt. Gleichzeitig ist das Rohr 13 an der Stelle 19 radial eingedrückt, so dass sich der in Figur 4 dargestellte sichelförmige Querschnitt des Katheterlumens 14 ergibt.

Im Übergangsabschnitt 15 befindet sich eine Drahtwendel 20, deren eines Ende über das Ende des Rohres 11 geschoben ist und auf diesem gehalten und zentriert wird. Die Drahtwendel 20 besteht aus zahlreichen gegeneinanderliegenden Windungen. Sie ist imstande, Druck in Längsrichtung zu übertragen, erlaubt jedoch Biegungen. Zur Anpassung an den Übergang zwischen dem größeren Durchmesser der Drahtwendel 20 und dem kleineren Außendurchmesser des Rohres 11 ist ein Verstärkungsschlauch 21 vorgesehen, dessen Wandstärke sich zum proximalen Ende hin keilförmig verringert. Auf diese Weise wird ein gleitender Kräfteübergang zwischen dem Rohr 11 und der Drahtwendel 20 erreicht.

Das andere (distale) Ende der Drahtwendel 20 stößt stumpf gegen das Ende des Rohres 13, das einen größeren Durchmesser hat als das Rohr 11.

Der gesamte Übergangsabschnitt 15 ist mit einer schlauchförmigen Hülle 23 bedeckt, die aus einem Schrumpfschlauch besteht. Die Hülle 23 weist auch eine entsprechende Öffnung für den Durchtritt des Rohres 16 auf. Sie ist mit diesem Rohr im Übrigen verschweißt, ebenso wie auch der Verstärkungsschlauch 21 mit dem Rohr 11 und der Hülle 23 verschweißt ist.

Durch die Drahtwendel 20 erstreckt sich ein im Katheter längslaufender Versteifungsdraht 25. Dieser ist an dem distalen Ende der Drahtwendel 20 an der Stelle 26 durch Anschweißen fixiert. Der Versteifungsdraht 25 erstreckt sich bis in den proximalen Schaft 10 und den distalen Schaft 12 hinein. Er überragt den Übergangsabschnitt 15 an jedem Ende. Der innenliegende Versteifungsdraht 25 sorgt für einen guten Steifigkeitsübergang vom proximalen Schaft 10 über die Drahtwendel 20 hinaus bis jenseits des Endes 18 des Führungsdrahtlumens 17.

## Patentansprüche

1. Ballonkatheter mit einem schlauchförmigen proximalen Schaft (10), einem ein Führungsdrahtlumen (17) enthaltenden schlauchförmigen distalen Schaft (12) und einem den proximalen und den distalen Schaft verbindenden Übergangsabschnitt (15), an dem ein Ende (18) des Führungsdrahtlumens (17) seitlich austritt, wobei der Übergangsabschnitt (15) eine Drahtwendel (20) enthält,
**dadurch gekennzeichnet,**
**dass** die Drahtwendel (20) mit einem Versteifungsdraht (25) verbunden ist, wobei der Versteifungsdraht an beiden Enden über die Drahtwendel (20) hinausragt und sich an einem Ende bis in den proximalen Schaft (10) und am anderen Ende bis in den Bereich des Führungsdrahtlumens (17) erstreckt.

2. Ballonkatheter nach Anspruch 1, **dadurch gekennzeichnet, dass** die Windungen der Drahtwendel (20) im unbelasteten Zustand gegeneinander liegen.

3. Ballonkatheter nach Anspruch 1 oder 2, **dadurch gekennzeichnet, dass** die Drahtwendel (20) an ihrem einen Ende mit dem Versteifungsdraht (25) verschweißt ist.

4. Ballonkatheter nach einem der Ansprüche 1-3, **dadurch gekennzeichnet, dass** die Drahtwendel (20) hohl ist und ein durchgehendes Katheterlumen (14) begrenzt und außen eine abdichtende Hülle (23) trägt.

5. Ballonkatheter nach einem der Ansprüche 1-4, **dadurch gekennzeichnet, dass** die Drahtwendel (20) von einer aus einem Schrumpfschlauch bestehenden Hülle (23) umgeben ist.

6. Ballonkatheter nach einem der Ansprüche 1-5, **dadurch gekennzeichnet, dass** der Versteifungsdraht (25) an derjenigen Seite an der Drahtwendel (20) anliegt, die der Seite des Austrittsendes (18) des Führungsdrahtlumens (17) abgewandt ist.

7. Ballonkatheter nach einem der Ansprüche 1-6, **dadurch gekennzeichnet, dass** die Drahtwendel (20) über oder in ein Ende eines den proximalen Schafts (10) bildenden Rohres (11) gesteckt ist, so dass ein homogener Steifigkeitsübergang entsteht.

## Claims

1. A balloon catheter with a hose-shaped proximal shaft (10), a hose-shaped distal shaft (12) enclosing a guide wire lumen (17), and a transition section (15) connecting the proximal and distal shafts, an end (18) of the guide wire lumen (17) exiting laterally from said transition section, wherein the transition section (15) includes a wire coil (20),
**characterized in**
**that** the wire coil (20) is connected to a stiffening wire (25) that projects beyond the wire coil (20) at both ends and extends into the proximal shaft (10) at the one end and into the region of the guide wire lumen (17) at the other end.

2. The balloon catheter of claim 1, **characterized in that** the windings of the wire coil (20) abut each other in the unloaded state.

3. The balloon catheter of claim 1 or 2, **characterized in that** the wire coil (20) has one of its ends welded to the stiffening wire (25).

4. The balloon catheter of one of claims 1-3, **characterized in that** the wire coil (20) is hollow and defines a continuous catheter lumen (14) and has a sealing sleeve (23) on the outside.

5. The balloon catheter of one of claims 1-4, **characterized in that** the wire coil (20) is enclosed by a sleeve (23) which is a shrinkable hose.

6. The balloon catheter of one of claims 1-5, **characterized in that** the stiffening wire (25) abuts the wire coil (20) on the side averted from the side of the exit end (18) of the guide wire lumen (17).

7. The balloon catheter of one of claims 1-6, **characterized in that** the wire coil (20) is slipped over or into an end of a tube (11) forming the proximal shaft (10), so that a homogeneous transition in rigidity is formed.

## Revendications

1. Cathéter ballon avec une tige (10) tubulaire proximale, une tige (12) tubulaire distale contenant une lumière à fil de guidage (17) et un segment de transition (15) reliant les tiges proximale et distale, duquel sort latéralement une extrémité (18) de la lumière à fil de guidage (17), le segment de transition (15) contenant un fil hélicoïdal (20),
**caractérisé en ce que** le fil hélicoïdal (20) est relié à un fil raidisseur (25), le fil raidisseur dépassant aux deux extrémités du fil hélicoïdal (20) et s'étendant d'un côté jusque dans la tige proximale (10) et de l'autre côté jusque dans la zone de la lumière à fil de guidage (17).

2. Cathéter ballon selon la revendication 1,
**caractérisé en ce qu'**à l'état non chargé les spires du fil hélicoïdal (20) se situent les unes contre les autres.

3. Cathéter ballon selon la revendication 1 ou 2, **caractérisé en ce que** le fil hélicoïdal (20) est soudé par l'un de ses extrémités au fil raidisseur (25).

4. Cathéter ballon selon l'une quelconque des revendications 1 à 3, **caractérisé en ce que** le fil hélicoïdal (20) est creux et délimite une lumière à cathéter (14) traversante et porte à l'extérieur une enveloppe (23) étanche.

5. Cathéter ballon selon l'une quelconque des revendications 1 à 4, **caractérisé en ce que** le fil hélicoïdal (20) est entouré par une enveloppe (23) en forme de flexible rétractable.

6. Cathéter ballon selon l'une quelconque des revendications 1 à 5, **caractérisé en ce que** le fil raidisseur (25) est relié au côté du fil hélicoïdal (20) qui est détourné du côté de l'extrémité de sortie (18) de la lumière à fil de guidage (17).

7. Cathéter ballon selon l'une quelconque des revendications 1 à 6, **caractérisé en ce que** le fil hélicoïdal (20) est enfoncé sur ou dans une extrémité d'un tube (11) formant la tige proximale (10) pour ainsi obtenir une transitions de raideur homogène.
